# EUROPEAN PATENT APPLICATION

(11) **EP 4 082 446 A1**
(43) Date of publication of application: **02.11.2022**
(21) Application number: 22170055.2
(22) Date of filing: 26.04.2022
(51) Int. Cl.: A61B 17/04

(54) **KNOTLESS SUTURE ANCHORS**

(30) Priority: 27.04.2021 US 202117241719
(71) Applicant: Medos International Sarl, 2400 Le Locle (CH)
(72) Inventor: SPENCINER, David, North Attleboro, 02760 (US); PILOTTE, Zachary, Raynham, 02767 (US); WHITTAKER, Gregory R., Stoneham, 02180 (US); ZIYA SENGUN, Mehmet, Foster City, 94404 (US)
(74) Representative: Carpmaels & Ransford LLP

(57) **Abstract**

Methods and systems are provided for securing tissue to bone. A suture anchor system can include a cannulated suture anchor having at least one driven feature disposed in a lumen and a bone-engaging feature disposed on an external surface. A cannulated inserter member can have an elongate member with a lumen and can be disposed within the lumen of the anchor. The elongate member can have a drive feature that can engage the driven feature. The inner tube can be disposed within the lumen of the inserter member. The inner tube can have a lumen and a length greater than the anchor such that the inner tube extends distally beyond the anchor. A distal swivel member can have a lumen and a distal end having a suture separating feature. The distal swivel member can be mounted adjacent to the anchor and can be rotatable relative to the anchor.

## Description

### FIELD

The present disclosure relates generally to methods and devices for securing soft tissue to bone.

### BACKGROUND

Tearing of, or complete or partial detachment of ligaments, tendons and/or other soft tissues from their associated bones within the body are commonplace injuries. Such injuries can result from excessive stresses being placed on these tissues. By way of example, tissue tearing or detachment may occur as the result of an accident such as a fall, over-exertion during a work-related activity, or during the course of an athletic event. In the case of tearing or a partial or complete detachment of soft tissue from a bone, surgery is typically required to reattach the soft tissue (or a graft tissue) to the bone.

One method of repairing such a tear is to stitch it closed by passing a length of suture through the tissue and tying the suture. Suture can also be used in conjunction with one or more suture anchors to repair such tissue tears. Sutures can be fastened to suture anchors and to tissue using knots tied by the surgeon during a repair procedure, or using "knotless" devices and methods, where one or more anchors and one or more sutures can be connected and tensioned without the surgeon needing to tie knots during the surgery. Knotless anchoring is of particular utility for minimally invasive surgeries, such as endoscopic or arthroscopic repairs, where the surgeon remotely manipulates the suture at the surgical site using tools inserted through a small percutaneous incision, small diameter cannula, or an endoscopic tube, which can make the knot-tying process difficult and tedious.

It can be challenging to maintain the desired alignment and tension on the operative suture in the course of a surgical procedure to reattach soft tissue. Further, existing suture anchors used to insert the anchors into bone may have certain disadvantages that complicate their use and/or impose certain undesirable limits.

Accordingly, there is a need for improved devices and systems for attaching tissue to bone.

### SUMMARY

In one aspect, suture anchor system includes a cannulated suture anchor having at least one driven feature disposed in a lumen thereof and at least one bone-engaging feature disposed on an external surface thereof. The system also has a cannulated inserter member having an elongate member with a lumen extending therethrough and configured to be removably disposed within the lumen of the suture anchor. The elongate member has at least one drive feature disposed thereon that is configured to engage the driven feature. The system also includes an inner tube that is configured to be removably disposed within the lumen of the inserter member, the inner tube having a lumen extending therethrough and having a length greater than a length of the suture anchor such that a distal end of the inner tube extends distally beyond a distal end of the suture anchor. A distal swivel member is distal to the suture anchor and has a lumen extending therethrough and a distal end having at least one distal-facing suture separating feature. The distal swivel member is configured to be mounted distally adjacent to the suture anchor and to be rotatable relative to the suture anchor.

The suture anchor can vary in numerous ways. For example, the driven feature can be a female receptacle with a hexagonal shape.

The bone engaging feature can have various configurations. For example, the bone-engaging feature can be a helical thread. The bone-engaging feature can be a plurality of barbs.

The cannulated inserter member can have various configurations. For example, the cannulated inserter member can further comprise a handle at a proximal end thereof. The drive feature of the inserter member can comprise a male member having a hexagonal shape.

The suture anchor system can have various configurations. For example, the system can further comprise a stay suture removably disposed in the lumen of the inner tube such that a loop of suture extends from the distal end of the inner tube and two free suture limbs extend from a proximal end of the inner tube. The distal-facing suture separating feature can comprise at least one of castellations and toothed structures.

The distal swivel member can have various configurations. For example, the distal swivel member can be attached to the suture anchor. A circumferential groove can be formed in one of the distal swivel member and the suture anchor is configured to mate with a circumferential protrusion on the other and the distal swivel member of the suture anchor. The distal swivel member cannot be attached to the suture anchor. The distal swivel member can be configured to be removably mountable on the inner tube in a friction fit.

In another aspect, a method for attaching soft tissue to bone can include securing at least one operative suture to a soft tissue to be reattached to bone. Free suture limbs of the at least one operative suture pass through at least one stay suture loop, the stay suture loop extending from an inner tube disposed within a suture anchor construct having a inserter member with a lumen extending therethrough within which the inner tube is disposed. A cannulated suture anchor is mounted upon the inserter member, and a distal swivel member is disposed on the inner tube distally adjacent to the suture anchor. The stay suture is tensioned to close the stay suture loop such that the operative suture is trapped between a distal end of the distal swivel member and the stay suture loop, the free suture limbs of the operative suture being constrained by at least one distal-facing suture separating feature formed on the distal swivel member. The inner tube is placed within a bone hole adjacent the soft tissue to be reattached to bone. The operative suture is tensioned to bring the soft tissue into secure engagement with bone. The suture anchor is implanted into the bone hole to engage bone while maintaining the distal swivel member substantially non-rotatable such that the free suture limbs of the operative suture are maintained substantially separated from each other. The stay suture and inner tube are then removed.

The cannulated suture anchor can have various configurations. For example, the cannulated suture anchor can include at least one bone-engaging feature disposed on an external surface thereof. The step of implanting can comprise rotating the suture anchor or impacting the suture anchor with a distally directed force.

The inserter member can have various configurations. For example, the inserter member can comprise a male member having a hexagonal shape.

The stay suture loop can have various configurations. For example, the stay suture loop can be formed from a stay suture removably disposed in a lumen of the inner tube such that the stay suture loop extends from a distal end of the inner tube and two free suture limbs extend from a proximal end of the inner tube.

The distal swivel member can have various configurations. For example, the distal swivel member can be attached to the cannulated suture anchor. A circumferential groove can be formed in one of the distal swivel member and the cannulated suture anchor can be configured to mate with a circumferential protrusion on the other and the distal swivel member of the cannulated suture anchor. At least one distal-facing suture separating feature can comprise at least one of castellations and toothed structures.

### BRIEF DESCRIPTION OF THE DRAWINGS

The present disclosure will be more fully understood from the following detailed description taken in conjunction with the accompanying drawings, in which:
FIG. 1 is a cross-sectional view of a suture anchor system;
FIG. 2A is a perspective view of a distal swivel member of the suture anchor system of FIG. 1;
FIG. 2B is a cross-sectional view of a distal swivel member and suture anchor of the suture anchor system of FIG. 1;
FIGS. 3A-3F schematically illustrate steps in a method of using the surgical anchor system including the distal swivel member of FIG. 2A to reattach soft tissue to bone;
FIG. 4 is a perspective view of another suture anchor system;
FIGS. 5A-5C schematically illustrate steps in a method of using the surgical anchor system of FIG. 4 to reattach soft tissue to bone;
FIG. 6 is a cross-sectional view of the operative sutures passing through the suture anchor system of FIG. 4; and
FIGS. 7A-7C schematically illustrate further method steps using the surgical anchor system of FIG. 4 to reattach soft tissue to bone.

### DETAILED DESCRIPTION

Certain examples will now be described to provide an overall understanding of the principles of the structure, function, manufacture, and use of the devices and methods disclosed herein. One or more examples are illustrated in the accompanying drawings. Those skilled in the art will understand that the devices and methods specifically described herein and illustrated in the accompanying drawings are non-limiting and that the scope of the present invention is defined solely by the claims. The features illustrated or described in connection with one example may be combined with the features of other examples. Such modifications and variations are intended to be included within the scope of the present invention.

Further, in the present disclosure, like-named components generally have similar features, and thus within a particular example each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used. In addition, the terms "about" and "substantially" are defined as ranges based on manufacturing variations and variations over temperature and other parameters.

Successful soft tissue repair surgery, such as rotator cuff repair, requires that the suture anchor be securely engaged within bone to avoid migration and/or dislodgement, and that the operative suture attached to the soft tissue and maintained against the bone by the suture anchor resist migration when subjected to loads. The most common mode of failure is due to suture migration or slippage, which occurs when the load applied to the operative suture exceeds the force applied by the suture anchor to compress the operative suture against the bone in which the suture anchor is implanted. Suture migration occurs at lower loads when the sutures themselves are misaligned and overlap within the bone hole. This can be particularly disadvantageous when an area of the bone adjacent to one of more strands of suture is softer and/or less healthy. The suture anchor systems described herein provide anchor implantation system designs optimized to maintain alignment of the operative suture (e.g., by maintaining the suture in a desired position or with desired spacing between operative suture strands) during and following insertion of the suture anchor to avoid suture migration and overlap, while maintaining secure engagement of the anchor within bone.

In at least some of the described examples, a suture anchor system is provided that includes suture separating features that enable the strands of the operative suture to be separated and positioned around the anchor with sufficient spacing to prevent an undue degree of suture overlap while enabling the anchor to inserted into the bone to secure soft tissue to the bone using the operative suture. During the course of a surgical procedure utilizing the suture anchor systems described herein, which is typically conducted arthroscopically, a suture anchor is removably disposed on an elongate shaft of an inserter device for delivery to its implantation site where it will engage operative suture to maintain the operative suture in tension and thus reattach soft tissue to bone. The suture anchor system also includes a distal swivel member removably mounted on the inserter device distal to the suture anchor. The distal swivel member is mounted in such a way that it is rotatable independent of the suture anchor. As explained in more detail below, the distal swivel member includes at its distal end one or more suture separating features that assist in maintaining the suture strands in a desired position and/or with a suitable degree of separation.

The suture separating features of the distal swivel member can be any sort of distally protruding structure(s) or feature(s) that can assist in maintaining separation, spacing, or position of the operative suture strands. The suture separating features of the distal swivel member can be castellations, toothed structures, and/or other protruding features arranged at the distal-facing surface of the distal swivel member. In this way, the operative suture can be captured by the castellations or toothed structures while the suture anchor is being inserted to keep the operative suture in proper alignment and/or with sufficient spacing between strands to prevent any undesired repositioning or overlap of the suture strands as the anchor is screwed into or otherwise implanted into bone.

The distal swivel member can be attached to the suture anchor such a way that the distal swivel member is independently rotatable relative to the suture anchor, forming an implantable suture anchor construct. Independent rotation of the distal swivel member can be achieved in a variety of ways. For example, the distal swivel member need not be attached to the suture anchor. Instead, the distal swivel member can be removably mounted adjacent to the inserter device, as explained below, distally adjacent to the suture anchor. In this way, any rotation of the suture anchor will not result in rotation of the distal swivel member, thus maintaining a desired position of the suture strands as established by the suture separating features. It can be desirable to physically connect the distal swivel member and the suture anchor. In one example, a circumferential groove can be formed in one of the distal swivel member and the suture anchor, and the groove is be configured to mate with a circumferential protrusion (that is partially or fully circumferential) that is formed on the other of the distal swivel member and the suture anchor. In this way, while the distal swivel member and the suture anchor are joined, they can be axially separated by a force that dislodges the protrusion from the groove. However, the placement of the protrusion within the groove and the relative dimensions of the protrusion and the groove enables independent rotation of the distal swivel member relative to the suture anchor.

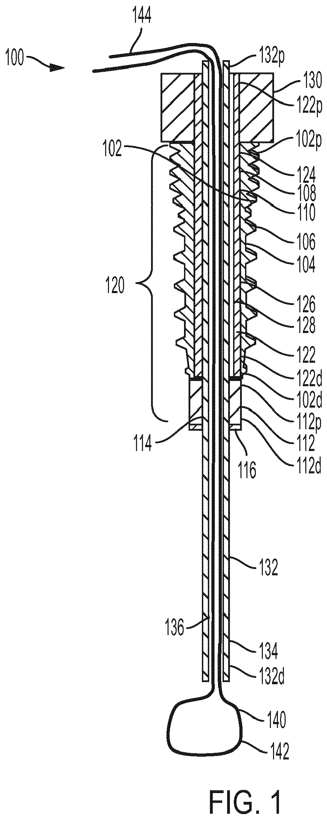

FIGS. 1-2B illustrate a suture anchor system 100 that includes a cannulated suture anchor 102 having a proximal end 102p, a distal end 102d, and an external surface 104 with a series of bone-engaging features 106. In this system, each of the plurality of longitudinally spaced bone-engaging features 106 can be formed at least partially circumferentially on the external surface 104 over at least a portion of a length of the suture anchor 102. The suture anchor 102 can have a lumen 108 extending through the suture anchor 102 from the proximal end 102p to the distal end 102d, with the lumen 108 being configured to removably secure the anchor 102 to an inserter device, as described in detail below. Although the lumen illustrated in FIG. 1 extends entirely through the anchor 102, a person skilled in the art will understand that the lumen 108 can terminate within the anchor 102 and not extend from the proximal end 102p to the distal end 102d.

The lumen 108 of the anchor 102 can have a driven feature 110 disposed within an internal surface of the lumen 108 that enables a rotational force to be imparted to the anchor 102, such as by a driver, as the anchor 102 is implanted in bone during a surgical procedure to reattach soft tissue to bone. While the driven feature 110 can take a variety of forms that will be understood by a person skilled in the art, in the system illustrated in FIG. 1, the driven feature 110 is a female receptacle with a hexagonal shape that is integral with the lumen 108 of the anchor 102. Alternative designs for the driven feature 110 can include but are not limited to a variety of other shapes, including an oval shape, a square shape, a pentagonal shape, etc.

The series of bone-engaging features 106 can be disposed over at least a portion of, and generally a majority of, a length of the external surface 104 of the anchor 102. Additionally, the series of bone-engaging features 106 can collectively encompass about 360° of a circumference of the anchor 102. While the bone-engaging features 106 can take a variety of forms that will be understood by a person skilled in the art, in the system illustrated in FIG. 1, the bone-engaging features 106 are in the form of a helical thread. As shown by way of example, the shape of the thread form may vary along the length of the suture anchor 102. Alternative designs for the bone-engaging features 106 can include but are not limited to a plurality of barbs, etc.

The suture anchor system 100 also includes a distal swivel member 112 having a proximal end 112p and a distal end 112d. The distal swivel member 112 can have a lumen 114 extending through the distal swivel member 112 from the proximal end 112p to the distal end 112d, with the lumen 114 being configured to removably secure the distal swivel member 112 to an inserter device, as described in detail below. The distal swivel member 112 is arranged distal to the suture anchor 102, with the proximal end 112p being adjacent to the distal end 102d. Additionally, the distal swivel member 112 can be configured to be rotatable relative to the suture anchor 102, as explained above. The distal swivel member 112 can be attached to the suture anchor 102.

As mentioned above, and illustrated in FIGS. 1-2B, the distal swivel member 112 can be attached to the suture anchor 102 such a way that the distal swivel member 112 is independently rotatable relative to the suture anchor 102, forming an implantable suture anchor construct 120. Independent rotation of the distal swivel member 112 can be achieved in a variety of ways. For example, the distal swivel member 112 need not be attached to the suture anchor 102. Instead, the distal swivel member 112 can be removably mounted adjacent to the inserter device, distally adjacent to the suture anchor 102, as illustrated in FIG. 1. In this way, any rotation of the suture anchor 102 will not result in rotation of the distal swivel member 112, thus maintaining a desired position of the suture strands as established by the suture separating features. In other examples, it can be desirable to physically connect the distal swivel member 112 and the suture anchor 102.

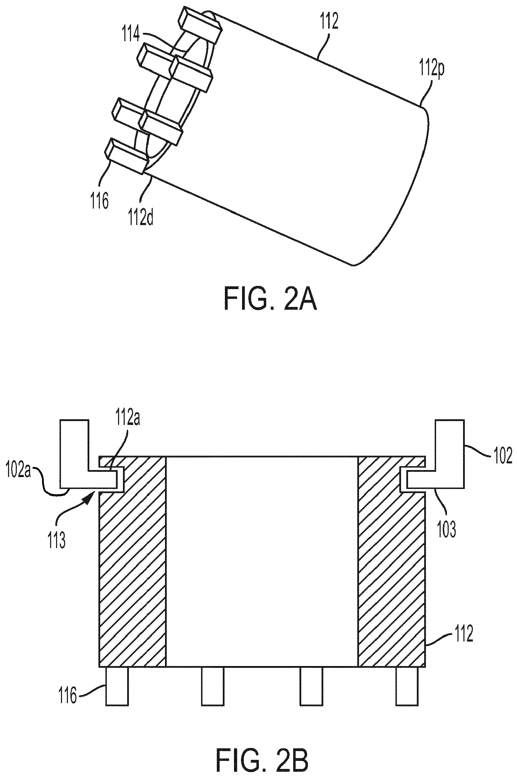

In one example, a circumferential groove 113 can be formed in one of the distal swivel member 112 and the suture anchor 102, and the groove 113 can be configured to mate with a circumferential protrusion 103 that is formed on the other of the distal swivel member 112 and the suture anchor 102. That is, a proximal facing face 112a of the distal swivel member 112 has one of a groove or a protrusion and a distal facing surface 102a of suture anchor 102 has the other of the groove or protrusion so that the two structures can mate to join the distal swivel member and the suture anchor in such a way that they are independently rotatable. In this way, while the distal swivel member 112 and the suture anchor 102 are joined, they can be axially separated by a force that dislodges the protrusion from the groove 113. However, the placement of the protrusion 103 within the groove 113 and the relative dimensions of the protrusion 103 and the groove 113 enables independent rotation of the distal swivel member 112 relative to the suture anchor 102. For example, as illustrated in FIG. 2B, the groove 113 is arranged on the distal swivel member 112, and the protrusion 103 is arranged on the suture anchor 102. The protrusion 103 can be flexible such that the protrusion 103 can deform to align or within or dislodge from the groove 113.

The suture separating features 116 of the distal swivel member 112 can be any sort of distally protruding structure(s) or feature(s) that can assist in maintaining separation, spacing, or position of the operative suture strands. An exemplary suture separating features 116 is illustrated in FIG. 2A, where the suture separating features 116 of the distal swivel member 112 can be castellations, toothed structures, and/or other protruding features arranged at the distal-facing surface of the distal swivel member. In this way, the operative suture can be captured by the castellations or toothed structures while the suture anchor 102 is being inserted to keep the operative suture in proper alignment and/or with sufficient spacing between strands to prevent any undesired repositioning or overlap of the suture strands as the anchor 102 is screwed into or otherwise implanted into bone.

In order to drive the anchor construct 120 into bone, an inserter device can be used to engage the driven feature 110 of the suture anchor 102. For example, a cannulated inserter member 122 can be removably disposed within the lumen 108 of the anchor 102. The cannulated inserter member 122 can have a proximal end 122p, a distal end 122d, and an external surface 124 with a drive feature 126 arranged on the external surface 124. The inserter member 122 can have a lumen 128 extending through the inserter member 122 from the proximal end 122p to the distal end 122d. Additionally, the inserter member 122 can include a handle 130 arranged at the proximal end 122p of the inserter member 122.

The drive feature 126 can be configured to engage the driven feature 110 of the suture anchor 102, enabling a rotational force to be applied to the anchor 102 as the anchor 102 is implanted in bone during a surgical procedure to reattach soft tissue to bone. While the drive feature 126 can take a variety of forms that will be understood by a person skilled in the art, in the system illustrated in FIG. 1, the drive feature 126 includes a male member having a shape (e.g., hexagonal) that is complementary to the shape of the driven feature 110. Alternative designs for the drive feature 126 can include but are not limited to a variety of other shapes, including an oval shape, a square shape, a pentagonal shape, etc..

As explained above, in some examples the suture anchor 102 is not secured to the distal swivel member 112. The anchor construct 120 can be formed with the aid of an additional alignment device that helps maintain the distal swivel member 112 in a position in which it abuts the suture anchor 102. For example, and as shown in FIG. 1, an inner tube 132 can be removably disposed within the lumen 128 of the inserter member 122 and the lumen 114 of the distal swivel member 112. The inner tube 132 can have a proximal end 132p, a distal end 132d, and an external surface 134. The external surface 134 is a smooth outer surface and is rotatable with respect to the suture anchor 102, the distal swivel member 112, and the inserter member 122. The inner tube 132 can have a lumen 136 extending through the inner tube 132 from the proximal end 132p to the distal end 132d. Additionally, the lumen 136 may have a smooth inner surface extending from the proximal end 132p to the distal end 132d. The inner tube 132 can be coterminous with the suture anchor 102 such that the distal swivel member 112 is mounted on the distal end of the inner tube 132. In such examples, the distal swivel member 112 can abut or be positioned such that distal swivel member 112 is close to abutting the distal end 102d of anchor 102.

The inner tube can have various configurations. For example, the inner tube 132 can have a length greater than a length of the anchor construct 120 such that a distal end 132d of the inner tube 132 extends distally beyond a distal end of the anchor construct 120. It is important to note that when the suture anchor 102 is connected to the distal swivel member 112 the use of the inner tube 132 can be optional. However, when the suture anchor 102 and the distal swivel member 112 are not connected to each other, the distal swivel member 112 is configured to be removably mountable on the inner tube 132 in a friction fit.

In addition to the suture separating feature of the distal swivel member 112, the suture anchor system 100 can further include a stay suture to aid in capturing and then aligning the operative sutures during insertion of the anchor construct 120. The stay suture 140 includes a loop 142 and free suture limbs 144. The stay suture 140 is removably disposed in the lumen 136 of the inner tube 132 such that a loop 142 of suture extends from the distal end 132d of the inner tube 132 and the two free suture limbs 144 extend from a proximal end 132p of the inner tube 132. The loop 142 is configured to be selectively tightened around operative sutures passing through the loop by tensioning one or both of the free suture limbs 144.

A person skilled in the art will appreciate that the suture anchor systems described herein can be used in a variety of surgical techniques to secure and/or reattach soft tissue to bone. The surgical procedure can be conducted as an open surgical procedure or as a minimally invasive surgical procedure, such as an arthroscopic procedure. Following preparation of the patient, an appropriate incision is made to access the surgical site. One or more holes are formed in bone as required by the particular surgical procedure, in proximity to the tissue repair site, to receive the one or more anchors to be used to anchor the soft tissue. One or more strands of operative suture are then passed through the detached tissue and then passed through a stay suture or positioned adjacent to the one or more suture anchors to be used in the procedure. The one or more suture anchors are then implanted into the corresponding bone hole(s) using an appropriate inserter tool as the operative sutures are appropriately tensioned to bring the detached tissue in proximity to bone. In so doing, the operative suture(s) are compressed within the bone hole between the anchor and the wall that defines the bone hole. Once the anchor is properly seated and the tissue is properly positioned adjacent to bone, the suture is tied off (either by cinching or tying a knot), any excess suture is removed, and the incision is closed.

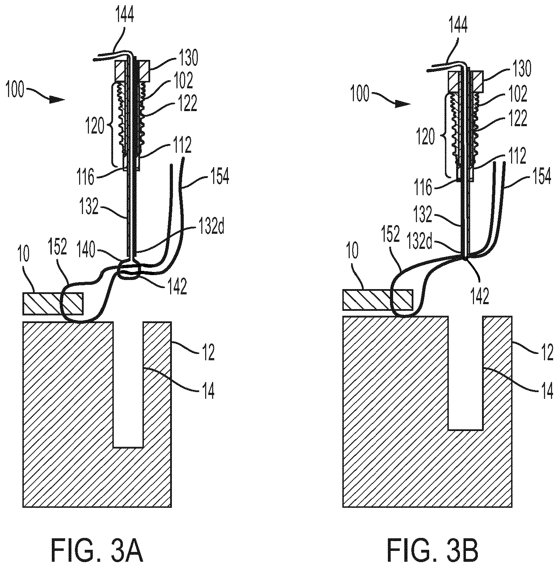

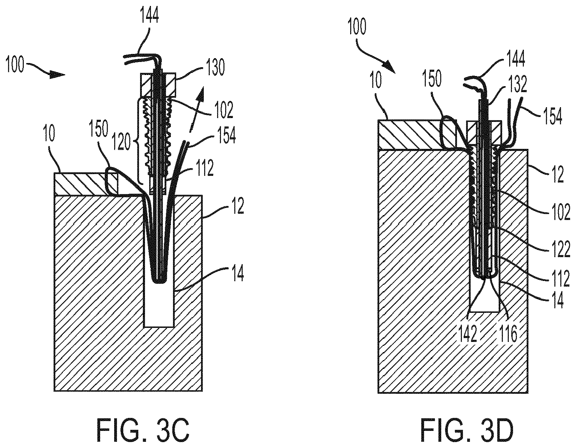

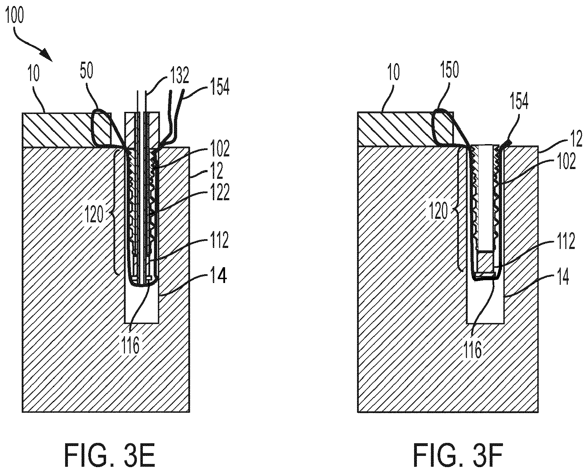

FIGS. 3A-3F illustrate the sequence of such a procedure in which the anchor construct 120 secures soft tissue 10 to bone 12 and the suture separating feature 116 promotes positioning of and/or prevents overlapping of the operative suture. The suture anchor system 100 can be fully assembled, in sterile packaging, prior to the beginning of the procedure. The anchor construct 120 can be removably disposed on the inner tube 132, with the cannulated inserter member 122 being inserted within the lumen of the suture anchor 102 such that rotational motion can be translated from the inserter member 122 to the anchor construct 120. In order to insert the construct 120, a bone hole 14 typically is first formed within a bone 12 in proximity to the soft tissue 10 that is being secured to the bone 12 by way of an operative suture. The stay suture 140, having a loop 142 and free ends 144, which is configured to secure the operative suture 150 during the procedure, is arranged within the lumen 136 of the inner tube of suture anchor system 100, as illustrated in FIG. 3A. When the one or more strands of operative suture 150 are positioned within the loop 142 of the stay suture 140 during a procedure, the loop 142 is closed by tensioning the stay suture, thus trapping the operative suture 150 between the distal end 132d of the inner tube 132 and the stay suture loop 142 as illustrated in FIG. 3B. The inner tube 132 is then disposed in the previously drilled bone hole 14 and the operative suture 150 is tensioned to the desired degree by pulling on the free ends 154, as illustrated in FIG. 3C. The anchor construct 120 can then be inserted into the bone hole 14 by applying a rotating or by impacting the suture anchor 102 with a distally directed force, as illustrated in FIG. 3D. With the anchor construct 120 being inserted into the bone hole, the free suture limbs 154 of the operative suture are constrained by the distal-facing suture separating feature 116 formed on the distal swivel member 112. The suture separating feature 116 maintains a desired position of the suture limbs 154 and also prevents the overlapping of the free suture limbs 154 during the insertion of the anchor construct 120. Since the distal swivel member 112 is rotatable with respect to the suture anchor 102, the rotational motion of the suture anchor 102 does not rotate the distal swivel member and thus enables the desired position of the operative suture limbs to be maintained. Once the anchor construct 120 is properly seated, the stay suture 140 is removed by pulling it out of the lumen 136, as illustrated in FIG. 3E. Additionally, the cannulated inserter member 122 and the inner tube 132 are removed leaving the anchor construct 120 within the bone hole 14, as illustrated in FIG. 3F. A person skilled in the art will understand that the positioning of the operative suture 150 between the walls of the bone hole and the anchor construct 120 maintains sufficient tension on the operative suture, and the suture separating features described herein reduce the chances of suture overlap.

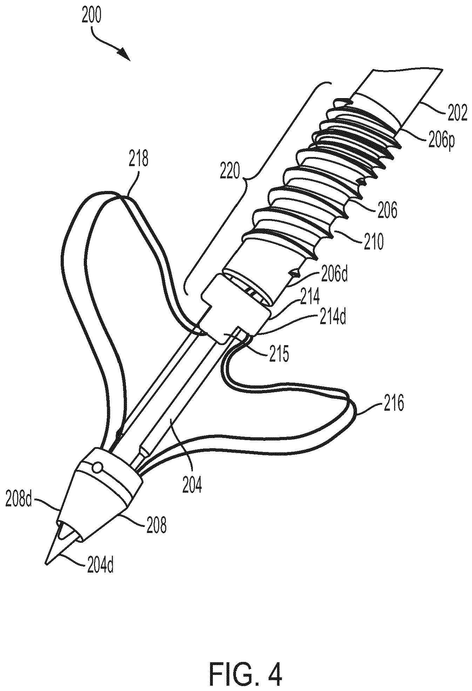

By way of non-limiting example, FIGS. 4-7C illustrate another suture anchor system 200 that includes and awl shaft and a dilator feature in combination with a distal swivel member.

Similar to the suture anchor system 100, FIGS. 4-7C illustrate a suture anchor system 200 that includes a driver shaft 202, an awl shaft 204, a suture anchor 206, a dilator feature 208, and a distal swivel member 214. The suture anchor 206, which can have external threads 210 formed thereon, has a lumen extending therethrough that removably receives the awl shaft 204. Similar to the anchor construct 120, the suture anchor 206 and the distal swivel member 214 can form an anchor construct 220. A distal driver feature of the driver shaft 202 is operably coupled to the suture anchor 206, as discussed in more detail below. The dilator feature 208, which is distal to the suture anchor 206, has a distal portion of the awl shaft 204 at least partially extending therethrough such that an awl of the awl shaft 204 extends distally from a distal end 208d of the dilator feature 208. While the suture anchor 206 can take a variety of forms, as explained herein, it can be a modification of the Healix Advance Self-Puncturing (HASP) suture anchor available from DePuy Synthes Mitek Sports Medicine of Raynham, MA

The suture anchor system 200 is used to initiate the formation of a hole in bone and, once the bone hole is formed, to drive the suture anchor into the hole. In some examples load is applied to the awl shaft 204 when the distal end 204d thereof is inserted into the bone. Thus, the awl shaft 204 acts to allow the system 200 to operate as a self-punching shaft used to initiate a hole in the bone without the need to use additional instruments to initiate the hole. Once the hole in the bone is initiated, the distal end 204d of the awl shaft 204 can be driven further into the hole by applying further load to the awl shaft 204. The dilator feature 208, which can be implantable, can assist in widening the hole in the bone as the hole is being formed. Once the bone hole is completely formed, the distal end 204d of the awl shaft 204 remains in the bone hole and the anchor construct 220 can be driven over the awl shaft 204.

The suture anchor 206 can have various configurations. In the illustrated system, as mentioned above, the suture anchor 206 has at least one external thread 210 formed thereon that is configured to engage the suture anchor 206 with the bone. However, the suture anchor 206 can have any suitable configuration and can have other bone-engaging features. The suture anchor can be a push-in style suture anchor.

Similar to the cannulated inserter member 122, a distal driver member can be configured to releasably mate with the suture anchor 206 and to thereby drive the suture anchor 206 mated thereto distally into bone (over the awl shaft 204), as discussed in more detail below. As illustrated herein, the distal driver member can be in the form of a male feature configured to be received within a corresponding female drive feature formed on at least a portion of an interior wall defining the lumen of the suture anchor 206.

The dilator feature 208 can be distally tapered and it can be in the form of a truncated cone or pyramid. The dilator feature 208 can be press-fit onto or otherwise releasably coupled with the awl shaft 204 and it can have any suitable dimensions. Furthermore, the dilator feature 208 can be implantable and it can be made from a non-metallic material, such as a bioabsorbable polymer.

As mentioned above, and illustrated in FIG. 4, the distal swivel member 214 can be attached to the suture anchor 206 in such a way that the distal swivel member 214 is independently rotatable relative to the suture anchor 206, forming an implantable suture anchor construct 220. Independent rotation of the distal swivel member 214 can be achieved in a variety of ways. For example, the distal swivel member 214 need not be attached to the suture anchor 206. Instead, the distal swivel member 214 can be removably mounted adjacent to the inserter device, distally adjacent to the suture anchor 206, similar to the suture anchor 102 and distal swivel member 112. In this way, any rotation of the suture anchor 206 will not result in rotation of the distal swivel member 214, thus maintaining a desired position of the suture strands as established by the suture separating features. It can be desirable to physically connect the distal swivel member 214 and the suture anchor 206.

In one example, a circumferential groove can be formed in one of the distal swivel member 214 (e.g., a proximal facing surface thereof) and the suture anchor 206 (e.g., a distal facing surface thereof) and the groove can be configured to mate with a circumferential protrusion that is formed on the other of the distal swivel member 214 and the suture anchor 206, similar to the to the suture anchor 102 and distal swivel member 112 described above with respect to FIG. 2B. In this way, while the distal swivel member 214 and the suture anchor 206 are joined, they can rotate independent of each other and be axially separated by a force that dislodges the protrusion from the groove.

The distal swivel member 214 can include a distal-facing suture separating feature 215 arranged on the distal end 214d of the distal swivel member 214. The suture separating features 215 of the distal swivel member 214 can be any sort of distally protruding structure(s) or feature(s) that can assist in maintaining separation, spacing, or position of the operative suture strands. An example of the suture separating features 215 is illustrated in FIGS. 4-5C, in which the suture separating features 215 of the distal swivel member 214 can be castellations, toothed structures, and/or other protruding features arranged at the distal-facing surface of the distal swivel member. In this way, the operative suture can be captured by the castellations or toothed structures while the suture anchor 206 is being inserted to keep the operative suture in proper alignment and/or with sufficient spacing between strands to prevent any undesired repositioning or overlap of the suture strands as the anchor 206 is screwed into or otherwise implanted into bone.

As further illustrated, stay suture limbs 216, 218 are passed through the lumen of the suture anchor 206 (which has the awl shaft 204 extending therethrough) and alongside the awl shaft 204 and the driver shaft 202 to manage the sutures running the length of the device. The stay suture limbs 216, 218 are also passed through the dilator feature 208 to form a closed loop with suture limbs 216, 218.

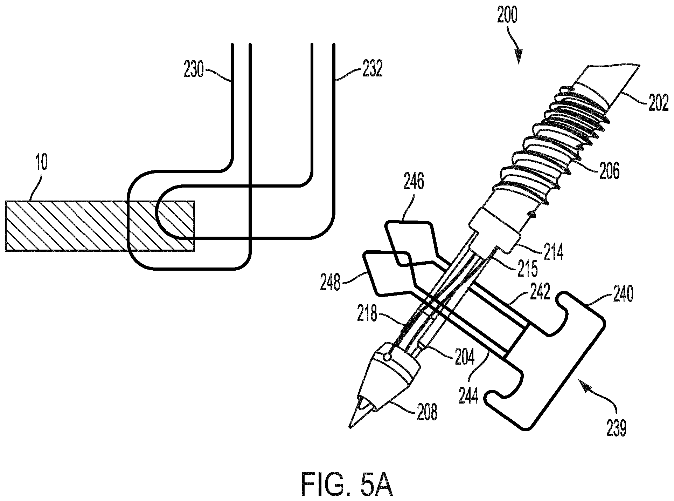

FIGS. 5A-7C illustrate a method of performing a surgical repair involving use of a suture anchor system, such as the suture anchor system 200 (shown by way of example only), to attach soft tissue 10 (e.g., tendon) to bone 12. It should be appreciated that the surgical repair method can be performed using other surgical systems, including surgical systems in which one or more components can be different from those included in the suture anchor system 200.

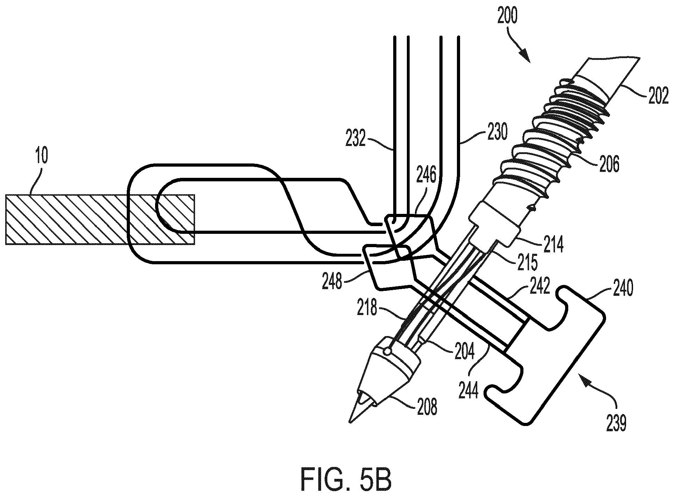

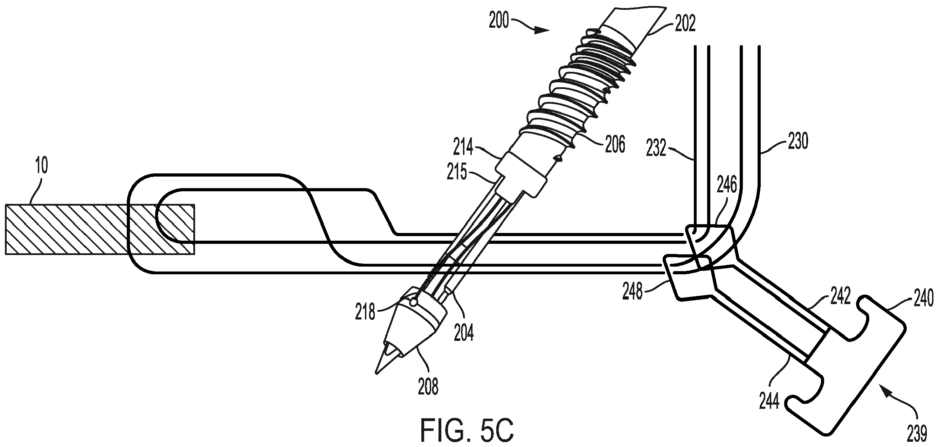

Prior to placing the awl shaft 204 into a bone hole, the operative sutures 230, 232 (which can be one or more suture strands) are secured to the soft tissue 10. Additionally, the operative sutures 230, 232 are passed through the stay suture limbs 216, 218 to associate the operative sutures. The operative sutures 230, 232 can be threaded through the stay sutures 216, 218 with the use of a suture threader tab 239 having a handle 240 having extensions 242, 244 and kites 246, 248. As shown in FIG. 5B, the operative suture strands are passed through the detached tissue 10 and then threaded through kites 246, 248. The threader tab 239 is then pulled, such as via handle 240, as shown in FIGS. 5B and 5C, to position the operative suture strands 230, 232 alongside the awl shaft 204 and within the stay suture limbs 216, 218.

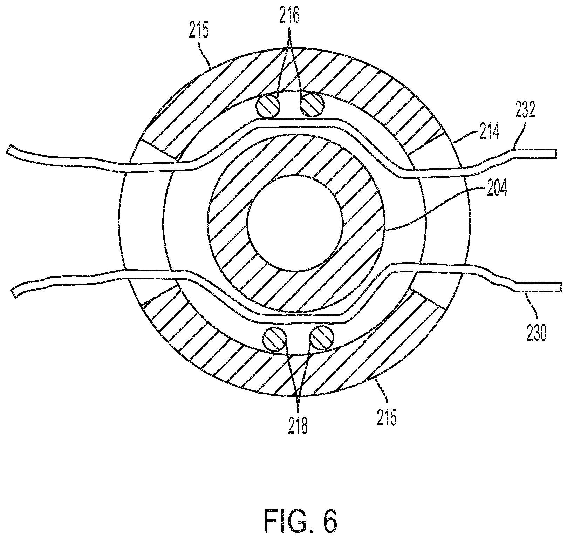

FIG. 6 illustrates the operative sutures 230, 232 arranged between the awl shaft 204 and the stay sutures 216, 218. In such a position the stay sutures 216, 218 can be tensioned to further encapsulate the operative sutures 230, 232 between the stay sutures 216, 218 and the awl shaft 204. Additionally, the operative sutures 230, 232 are arranged such that as the anchor construct 220 is rotated into a bone hole, the suture separating feature 215 will prevent overlapping of the operative sutures 230, 232 and help maintain a desired position of the operative sutures 230, 232. With the operative sutures 230, 232 properly arranged within the stay sutures 216, 218, the operative sutures 230, 232 can be separated from the kites 246, 248.

After the operative sutures 230, 232 are properly contained by the stay sutures 216, 218, the awl of the awl shaft 204 is inserted into the bone 12 to initiate a hole 14 in the bone 12 at a desired location similar to the method illustrated in FIGS. 3A-3F. Tension is applied to the operative sutures 230, 232 after the distal end 204d of the awl shaft 204 is inserted into the bone 12 to form the hole. In some examples, the awl shaft 204 is a self-punching shaft configured to initiate and create the hole in the bone such that no additional instrument is required. Once the hole in the bone is initiated, the awl shaft 204 is driven further distally into the bone 12 to completely form the bole hole 14. In particular, a suitable instrument, such as mallet, hammer, or other impactor, is used to apply force to the awl shaft 204 to thereby cause the awl shaft 204 to drive distally into the bone 12. As the awl shaft 204 is driven distally into the bone 12, the dilator feature 208 widens the hole 14.

The awl of the awl shaft 204 is driven into bone 12 such that the dilator feature 208 coupled thereto is inserted into the hole 14 in the bone. A portion of the awl shaft 204 is also inserted into the hole, with the suture anchor 206 is positioned at a desired position relative to the bone hole 14.

Once the suture anchor system 200 is moved from the bone hole forming configuration to the suture anchor driving configuration, the suture anchor 206 can be driven into the bone hole 14. Thus, the driver shaft 202 is rotated to cause the suture anchor 206 to rotate and to drive the suture anchor 206 and the distal swivel member 214 distally to the bone hole.

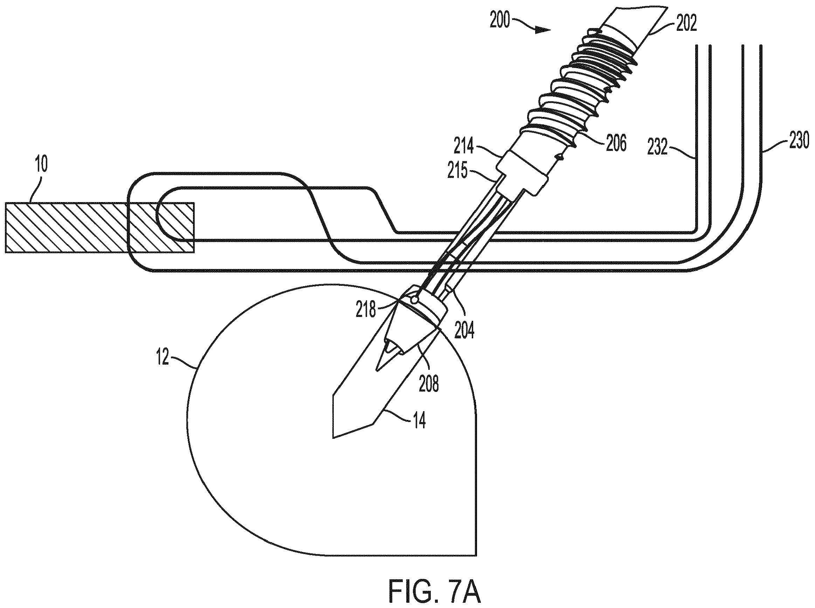

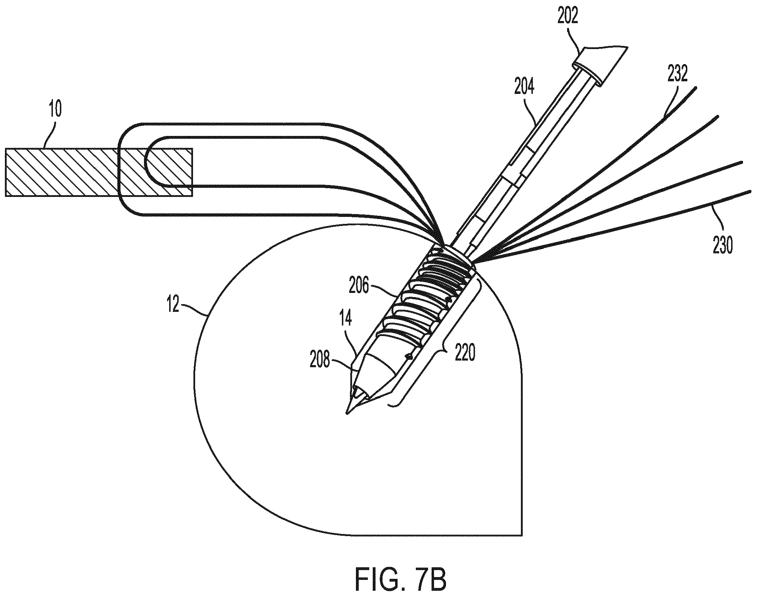

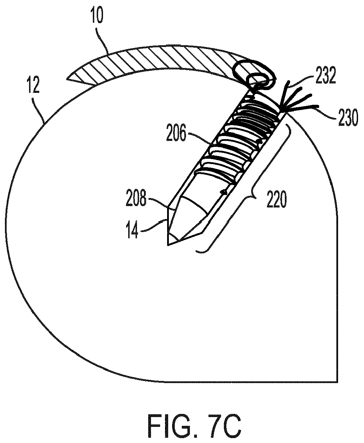

As the anchor construct 220 is advanced into the hole, the threads 210 of the suture anchor 206 engage the bone. Additionally, the suture separating feature 215 of the distal swivel member 214 traps the operative sutures 230, 232 and prevent overlapping and misalignment. As the driver shaft 202 is rotated, the awl shaft 204, which extends through a lumen of the driver shaft 202 and through the dilator feature 208, remains stationary. As explained above, the dilator feature 208 is independently rotatable relative to the suture anchor 206 and likewise does not rotate as the suture anchor 206 is rotated, helping to maintain the desired position of the suture strands. The rotation of the driver shaft 202 causes the anchor construct 220 to move distally towards the distal dilator feature 208 to contact the distal dilator feature 208 in the bone hole 14, causing the operative sutures 230, 232 to be secured between an interior wall of the bone hole 14 and an outer surface of the suture anchor 206. As illustrated in FIGS. 7B-7C, as the suture anchor 206 is moved distally, the distal swivel member 214 is also moved distally towards the distal dilator feature 208. In order to fully insert the suture anchor 206, the distal swivel member 214 can slide into a space arranged between the dilator feature 208 and the awl shaft 204. This arrangement of the dilator feature 208 sliding over the distal swivel member 214 allows the suture anchor 206 to contact the dilator feature 208 when fully inserted. Once the anchor construct 220 has been driven into the bone hole 14, the driver shaft 202, as well as the awl shaft 204 seated therein, can be separated from the anchor construct 220. The dilator feature 208 and the anchor construct 220 with the operative sutures 230, 232 coupled thereto remain implanted in the bone hole 14, thereby attaching the tissue 10 to the bone 12.

The methods and systems described herein can have different variations. For example, multiple sutures can be used to couple tissue to bone. Also, one or more operative sutures can be loaded within the anchor before or during a surgical procedure. For example, a suture anchor can have at least one operative suture pre-loaded thereto such that the suture anchor includes the suture.

Furthermore, the anchor may be pre-loaded on the inserter shaft.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device, e.g., the shafts, can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the components of the system described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred the components are sterilized. This can be done by any number of ways known to those skilled in the art including beta or gamma radiation, ethylene oxide, steam, and a liquid bath (e.g., cold soak).

One skilled in the art will appreciate further features and advantages of the described subject matter. Accordingly, the present disclosure is not to be limited by what has been particularly shown and described, except as indicated by the appended claims.

The following relate to numbered aspects of the invention
1. A suture anchor system, comprising:
   a cannulated suture anchor having at least one driven feature disposed in a lumen thereof and at least one bone-engaging feature disposed on an external surface thereof;
   a cannulated inserter member having an elongate member with a lumen extending therethrough and configured to be removably disposed within the lumen of the suture anchor, the elongate member having at least one drive feature disposed thereon that is configured to engage the driven feature;
   an inner tube configured to be removably disposed within the lumen of the inserter member, the inner tube having a lumen extending therethrough and having a length greater than a length of the suture anchor such that a distal end of the inner tube extends distally beyond a distal end of the suture anchor; and
   a distal swivel member distal to the suture anchor and having a lumen extending therethrough and a distal end having at least one distal-facing suture separating feature, the distal swivel member being configured to be mounted distally adjacent to the suture anchor and to be rotatable relative to the suture anchor.
2. The suture anchor system of aspect 1, wherein the driven feature is a female receptacle with a hexagonal shape.
3. The suture anchor system of aspect 1, wherein the bone engaging feature is a helical thread.
4. The suture anchor system of aspect 1, wherein the bone engaging feature is a plurality of barbs.
5. The suture anchor system of aspect 1, wherein the cannulated inserter member further comprises a handle at a proximal end thereof.
6. The suture anchor system of aspect 2, wherein the drive feature of the inserter member comprises a male member having a hexagonal shape.
7. The suture anchor system of aspect 1, further comprising a stay suture removably disposed in the lumen of the inner tube such that a loop of suture extends from the distal end of the inner tube and two free suture limbs extend from a proximal end of the inner tube.
8. The suture anchor system of aspect 1, wherein the distal swivel member is attached to the suture anchor.
9. The suture anchor system of aspect 8, wherein a circumferential groove formed in one of the distal swivel member and the suture anchor is configured to mate with a circumferential protrusion on the other and the distal swivel member of the suture anchor.
10. The suture anchor system of aspect 1, wherein the distal swivel member is not attached to the suture anchor.
11. The suture anchor system of aspect 1, wherein the distal swivel member is configured to be removably mountable on the inner tube in a friction fit.
12. The suture anchor system of aspect 1, wherein the distal-facing suture separating feature comprises at least one of castellations and toothed structures.
13. A method for attaching soft tissue to bone, comprising:
   securing at least one operative suture to a soft tissue to be reattached to bone;
   passing free suture limbs of the at least one operative suture through at least one stay suture loop, the stay suture loop extending from an inner tube disposed within a suture anchor construct having a inserter member with a lumen extending therethrough within which the inner tube is disposed, a cannulated suture anchor mounted upon the inserter member, and a distal swivel member disposed on the inner tube distally adjacent to the suture anchor;
   tensioning the stay suture to close the stay suture loop such that the operative suture is trapped between a distal end of the distal swivel member and the stay suture loop, the free suture limbs of the operative suture being constrained by at least one distal-facing suture separating feature formed on the distal swivel member;
   placing the inner tube within a bone hole adjacent the soft tissue to be reattached to bone;
   tensioning the operative suture to bring the soft tissue into secure engagement with bone;
   implanting the suture anchor into the bone hole to engage bone while maintaining the distal swivel member substantially non-rotatable such that the free suture limbs of the operative suture are maintained substantially separated from one another; and
   removing the stay suture and inner tube.
14. The suture anchor system of aspect 13, wherein the cannulated suture anchor includes at least one bone-engaging feature disposed on an external surface thereof.
15. The suture anchor system of aspect 13, wherein the inserter member comprises a male member having a hexagonal shape.
16. The suture anchor system of aspect 13, wherein the stay suture loop is formed from a stay suture removably disposed in a lumen of the inner tube such that the stay suture loop extends from a distal end of the inner tube and two free suture limbs extend from a proximal end of the inner tube.
17. The suture anchor system of aspect 13, wherein the distal swivel member is attached to the cannulated suture anchor.
18. The suture anchor system of aspect 17, wherein a circumferential groove formed in one of the distal swivel member and the cannulated suture anchor is configured to mate with a circumferential protrusion on the other and the distal swivel member of the cannulated suture anchor.
19. The suture anchor system of aspect 13, wherein the step of implanting comprises rotating the suture anchor or impacting the suture anchor with a distally directed force.
20. The suture anchor system of aspect 13, wherein the at least one distal-facing suture separating feature comprises at least one of castellations and toothed structures.

## Claims

1. A suture anchor system, comprising:
a cannulated suture anchor having at least one driven feature disposed in a lumen thereof and at least one bone-engaging feature disposed on an external surface thereof;
a cannulated inserter member having an elongate member with a lumen extending therethrough and configured to be removably disposed within the lumen of the suture anchor, the elongate member having at least one drive feature disposed thereon that is configured to engage the driven feature;
an inner tube configured to be removably disposed within the lumen of the inserter member, the inner tube having a lumen extending therethrough and having a length greater than a length of the suture anchor such that a distal end of the inner tube extends distally beyond a distal end of the suture anchor; and
a distal swivel member distal to the suture anchor and having a lumen extending therethrough and a distal end having at least one distal-facing suture separating feature, the distal swivel member being configured to be mounted distally adjacent to the suture anchor and to be rotatable relative to the suture anchor.

2. The suture anchor system of claim 1, wherein the driven feature is a female receptacle with a hexagonal shape.

3. The suture anchor system of claim 1 or 2, wherein the bone engaging feature is a helical thread.

4. The suture anchor system of claim 1 or 2, wherein the bone engaging feature is a plurality of barbs.

5. The suture anchor system of any of claims 1 to 4, wherein the cannulated inserter member further comprises a handle at a proximal end thereof.

6. The suture anchor system of claim 2, wherein the drive feature of the inserter member comprises a male member having a hexagonal shape.

7. The suture anchor system of any of claims 1 to 6, further comprising a stay suture removably disposed in the lumen of the inner tube such that a loop of suture extends from the distal end of the inner tube and two free suture limbs extend from a proximal end of the inner tube.

8. The suture anchor system of any of claims 1 to 7, wherein the distal swivel member is attached to the suture anchor.

9. The suture anchor system of claim 8, wherein a circumferential groove formed in one of the distal swivel member and the suture anchor is configured to mate with a circumferential protrusion on the other and the distal swivel member of the suture anchor.

10. The suture anchor system of any of claims 1 to 7, wherein the distal swivel member is not attached to the suture anchor.

11. The suture anchor system of any of claims 1 to 7, wherein the distal swivel member is configured to be removably mountable on the inner tube in a friction fit.

12. The suture anchor system of any of claims 1 to 11, wherein the distal-facing suture separating feature comprises at least one of castellations and toothed structures.

13. The suture anchor system of any preceding claim for use in a method of attaching soft tissue to bone.
